# EUROPEAN PATENT SPECIFICATION

(11) **EP 0 836 498 B1**
(45) Date of publication and mention of the grant of the patent: **01.09.2004**
(21) Application number: 96923220.6
(22) Date of filing: 06.06.1996
(51) Int. Cl.: A61M 16/00, A62B 9/06, A61M 16/04

(54) **IMPROVED TRACHEOSTOMY TUBES**
VERBESSERTER LUFTRÖHRENSCHLAUCH
AMELIORATION APPORTEE A UNE CANULE DE TRACHEOTOMIE

(30) Priority: 07.06.1995 US 476005
(43) Date of publication of application: 22.04.1998
(73) Proprietor: MALLINCKRODT INC., St. Louis, MO 63134 (US)
(72) Inventor: PRICHARD, Cheryl, B., St. Peters, MO 63376 (US); TAYLOR, Geraldine, Ballinaloe County Galway (IE); MAHONEY, Michael, R., Lake St. Louis, MO 63367 (US)
(74) Representative: Carpmaels & Ransford
(86) International application number: PCT/US1996/009185
(87) International publication number: WO 1996/040339

(56) References cited:
- US-A- 5 067 497
- US-A- 5 311 864

## Description

The present invention relates to tracheostomy tubes which are used as an alternate airway passage for patients. In particular, the present invention relates to tracheostomy tubes which have an additional lumen and methods of making them.

Tracheostomy tubes have been used for some time.to provide a bypass supply of air or mixture of gases to a patient having an obstruction in the larynx or the pharynx area of the throat. The distal end of the tracheostomy tube is inserted through an incision or stoma in the patient's neck below the obstructed area. An inflatable cuff near the distal end of the tracheal tube is inflated securely to position the tube within the trachea of the patient. The proximal end of the tracheostomy tube remains outside the trachea in communication with ambient air or with a ventilator and permits passage of air into the trachea.

Tracheostomy tubes are often used following a surgical procedure in the larynx or pharynx to aid the patient in breathing during the surgical recovery period. In these cases, the tracheostomy tube is intended to be used for a relatively short period of time until the patient can resume breathing normally. In other cases, tracheostomy tubes are used for extended periods of time or may be permanent.

In all cases, it is necessary periodically to clean the area of the tube above the cuff of the tracheostomy tubes of mucus and other secretions which build up in the trachea.

When tracheostomy tubes are used, it is often desirable to have an additional lumen, wherein the main lumen provides the airway passage and the additional lumen provides a means for introduction of medicants and a means for suctioning of secretions which may collect in the patient's airway above the cuff. The inclusion of an additional lumen is important, as it allows for suctioning, while maintaining the main lumen for breathing. This is especially important when the normal airway passages of the patient are completely blocked.

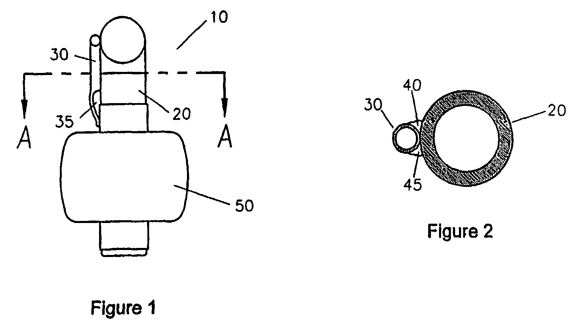

Prior art tracheostomy tubes having an additional lumen have been comprised of two tubes, each having a cylindrical cross-section, bonded together. Such a prior art tube is shown in Figs. 1 and 2 wherein the tracheostomy tube, generally designated by reference numeral 10, comprises a first tube 20, bonded to a second tube 30. The tube 10 includes an inflatable cuff 50, formed near the distal end and surrounding the first tube 20. The second tube 30, extends to just above the cuff 50, and includes openings 35, which may be used to admit medicants above the cuff 50, or to suction material from above the cuff 50. Prior art tubes of this type have a number of disadvantages, including the use of two cylindrical tubes 20, 30, which creates a tracheostomy tube having a relatively large and non-symmetrical overall cross-section. This can create discomfort for the patient, especially in the stoma area. Further, as best shown in Fig. 2, the first tube 20, and second tube 30, must be bonded together by bond seams 40, 45, which run the entire length of the second tube 30. The bond seams 40, 45, necessarily are wedge shaped to create a sufficient bond between the tubes 20, 30, and to present a more even and consistent outside diameter for the tracheostomy tube 10. However, the bond seams 40, 45, can still cause discomfort to the patient and add considerably to the expense and processing required to form the tracheostomy tube.

Therefore, there remains a need in the art for improvements to double lumen tracheostomy tubes.

It is one object of the present invention to provide a lower profile two lumen tracheostomy tube.

It is a further object of the present invention to provide a two lumen tracheostomy tube which can be more easily manufactured than those of the prior art.

The above objects and others, are accomplished according to the present invention by providing a tracheostomy tube comprised of a first tube having a circular cross-section bonded to a second tube initially having a "D" shaped external cross-section, and a lumen with a substantially circular cross section.

### Brief Description of The Drawings

Fig. 1 is a plan view of a tracheostomy tube as known in the prior art.
Fig. 2 is a cross sectional view taken along line A-A of Fig. 1, of a tracheostomy tube as known in the prior art.
Fig. 3 is a plan view of a tracheostomy tube according to one embodiment of the present invention.
Fig. 4 is a cross sectional view taken along line B-B of Fig. 3, of a tracheostomy tube according to one embodiment of the present invention.
Fig. 5 is a cross sectional view of the "D" shaped second tube of the tracheostomy tube according to the present invention.
Fig. 6A and 6B are cross sectional views showing progressive stages of the manufacture of a tracheostomy tube according to one embodiment of the present invention.

### Detailed Description of The Invention

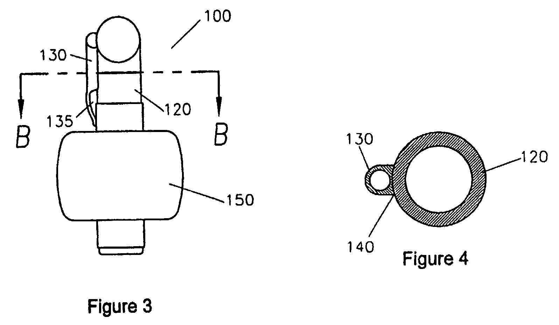

Fig. 3 is a plan view of a tracheostomy tube generally designated by reference numeral 100, according to one embodiment of the present invention. In particular, tracheostomy tube 100, comprises a first tube 120, having a generally circular cross-section, and a second tube 130, having a D-shaped external cross-section, bonded to the first tube 120. The tube 100 includes an inflatable cuff 150, formed near the distal end and surrounding the first tube 120. The second tube 130 extends to just above the cuff 150, and includes openings 135 which may be used to admit medicants above the cuff 150, or to suction material from above the cuff 150.

Fig. 4 is a cross sectional view taken along line B-B of Fig. 3, of a tracheostomy tube according to the present invention, showing the overall profile comprising first tube 120, and second tube 130, bonded together along seam 140.

As will be apparent from a review of Figs. 3 and 4, the tracheostomy tube 100, according to the present invention overcomes all of the disadvantages noted above with respect to the prior art. In particular, tracheostomy tube 100 presents a much smaller overall cross-section which reduces discomfort for the patient, especially in the stoma area. Further, the seam 140 is much smaller and less intrusive, virtually eliminating one source of discomfort for the patient. The seam 140 actually comprises only a thin layer of adhesive between the tubes 120, 130, but achieves a greater binding strength therebetween than with the wedge shaped seams 40, 45 of the prior art.

As can be seen in Figs. 3 and 4, first tube 120 has a substantially larger cross sectional area than that of second tube 130. Any appropriate sized tubes may be used according to the present invention, but sizes should be picked to present the lowest overall profile which still maintains the desired air supply capabilities through the first tube 120, and provides the desired medicant delivery or suctioning capabilities through the second tube 130. In particular, first tube 120 may be of any standard size used for tracheostomy tubes, e.g. having inside diameters in the range of 5.0 mm to 10.0 mm and outside diameters in the range of 7.0 mm to 13.3 mm. The second tube 130 may have the same size for all uses and has an inside diameter of about 0.075 mm to 0.08 mm. The outside diameter of the second tube 130 has two dimensions, the first extending from the flat side to the curved side of the D shape and being about 0.128 mm, and the second extending from side to side of the D shape and being about 0.143 mm.

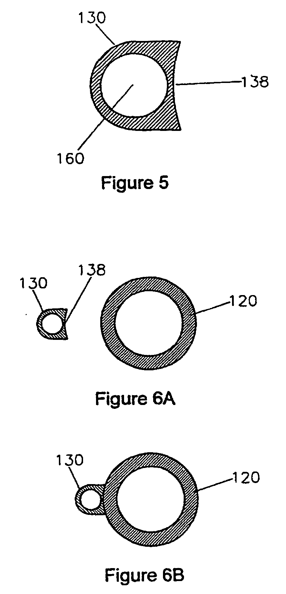

Fig. 5 is a cross sectional view of a second tube 130, as described above with reference to Figs. 3 and 4. The second tube 130 initially has a "D" shaped profile which provides a bonding surface 138 for bonding to the exterior of the first tube 120 (Figs. 3 and 4). It is noted that the lumen 160 of the second tube 130, is not compromised by the "D" shape profile, but rather retains a substantially circular cross section. This is important in providing adequate flow rates of medicants, and to allow for adequate suctioning of material from above the cuff.

As best shown in Figs. 6A and 6B, the process of making a tracheostomy tube according to the present invention is simplified and improved. Fig. 6A shows a first step wherein tubes 120 and 130 are initially present as two separate tubes. It will be recognized that tube 130 has a D-shaped external cross-section. An adhesive may be applied to one or both outer surfaces on tubes 120, 130, which will create the seam 140 (Fig. 4). To avoid an excess of adhesive being applied, the adhesive is preferably applied only to the bonding surface 13 8 of the D-shaped tube 130.

The tubes 120, 130, are then attached to each other as shown in Fig. 6B by any suitable bonding procedure, such as solvent or chemical bonding, during which the D-shaped tube 130 conforms to the shape of the cylindrical tube 120.

As noted above, the lumen of the "D" shaped tube retains a substantially circular cross section. Advantageously, it has been found that the "D" shaped second tube according to the present invention actually allows greater flow rates or suctioning rates than provided by the prior art, while still presenting the lower profile. This can be extremely advantageous when it is necessary to supply medicants to or to suction secretions from the airway of the patient, because such can be done more quickly.

The tracheostomy tube of the present invention can be formed of any suitable material which provides enough stiffness to allow easy insertion into the trachea of the patient, and enough flexibility to avoid undue stress and damage to the trachea. For example, the tracheostomy tube may be advantageously formed of flexible plastic materials, such as plasticized polyvinyl chloride, polyurethane or silicone.

The individual tubes of the tracheostomy tube according to the present invention may be formed by extrusion techniques, although one skilled in the art will recognize that other methods of manufacture can also be carried out. As will further be understood by one skilled in the art, tracheostomy tubes will vary in size in order to accommodate different patients and clinical needs. The present invention is equally applicable to all sizes and shapes of tracheostomy tubes.

The foregoing has been a description of certain preferred embodiments of the present invention, but is not intended to limit the invention in any way. Rather, many modifications, variations and changes in details may be made within the scope of the present invention.

## Claims

1. A tracheostomy tube (100), comprising
a first tube (120) having a circular cross-section; and
a second tube (130) having a D-shaped external cross section;
wherein said first tube (120) and said second tube (130) are bonded together, and wherein the second tube (130) has a lumen (160) of substantially circular cross section.

2. A tracheostomy tube (100) according to claim 1, wherein said second tube (130) has a bonding surface (138) that conforms to the shape of the exterior of said first tube (120).

3. A tracheostomy tube (100) according to claim 1 or claim 2, wherein said first tube (120) has an inside diameter in the range of 5.0 mm to 10.0 mm, and said second tube (130) has an inside diameter in the range of 0.075 mm to 0.080 mm.

4. The tracheostomy tube (100) of any one of the preceding claims, comprising
an inflatable cuff (150) surrounding said first tube (120) near the distal end of said first tube (120);
the second tube (130) being bonded to said first tube (120) from the proximal end of the first tube (120) to just above said inflatable cuff (150), and having an opening formed at a location just above said cuff (150) which allows medicants, in use, to be delivered through said tracheostomy tube (100) for a patient and which also allows secretions, in use, to be suctioned out of a patient through said tracheostomy tube (100).

5. A method of making a tracheostomy tube (100), comprising providing a first tube (120) having a cylindrical cross-section;
providing a second tube (130) having a D-shaped external cross-section, such that said second tube (130) has a bonding surface (138) for bonding to the exterior of the first tube (120) and a rounded portion, said second tube (130) having a lumen (160) of substantially circular cross-section;
applying an adhesive on at least one portion of one of said first or second tube (130)s; and
attaching the bonding surface (138) of said second tube (130) to an exterior of said first tube (120) thereby bonding said second tube (130) to said first tube (120) such that said bonding surface (138) of said second tube (130), which conforms to exterior of said first tube (120), creates a bonding seam (140) between the two tubes (120, 130).

6. A method according to claim 5, wherein said step of applying an adhesive includes the step of applying an adhesive to said bonding surface (138) of said second tube (130).

## Patentansprüche

1. Tracheostomie-Tubus (100), mit
einem ersten Tubus (120) mit einem kreisförmigen Querschnitt; und
einem zweiten Tubus (130) mit einem D-förmigen Außenquerschnitt,
wobei der erste Tubus (120) und der zweite Tubus (130) miteinander verbunden sind, und wobei der zweite Tubus (130) ein Lumen (160) mit im Wesentlichen kreisförmigem Querschnitt aufweist.

2. Tracheostomie-Tubus (100) nach Anspruch 1, wobei der zweite Tubus (130) eine Verbindungsfläche (138) aufweist, die an die Form der Außenseite des ersten Tubus (120) angepasst ist.

3. Tracheostomie-Tubus (100) nach Anspruch 1 oder Anspruch 2, wobei der erste Tubus (120) einen Innendurchmesser im Bereich von 5,0 mm bis 10,0 mm und der zweite Tubus (130) einen Innendurchmesser im Bereich von 0,075 mm bis 0,080 mm aufweist.

4. Tracheostomie-Tubus (100) nach einem der vorhergehenden Ansprüche, mit einer aufblasbaren Manschette 150, die den ersten Tubus (120) in der Nähe des distalen Endes des ersten Tubus (120) umgibt;
wobei der zweite Tubus (130) mit dem ersten Tubus (120) vom proximalen Ende des ersten Tubus (120) bis gerade oberhalb der aufblasbaren Manschette (150) verbunden ist und eine Öffnung aufweist, die an einer Stelle gerade oberhalb der Manschette (150) gebildet ist, die es ermöglicht, im Gebrauch einem Patienten Medikamente durch den Tracheostomie-Tubus (100) zu verabreichen und es ebenso ermöglicht, im Gebrauch Sekrete aus dem Patienten durch den Tracheostomie-Tubus (100) hindurch abzusaugen.

5. Verfahren zum Herstellen eines Tracheostomie-Tubus (100), mit den Schritten: Bereitstellen eines ersten Tubus (120) mit einem zylindrischen Querschnitt;
Bereitstellen eines zweiten Tubus (130) mit einem D-förmigen Außenquerschnitt, so dass der zweite Tubus (130) eine Verbindungsfläche (138) zum Verbinden mit der Außenseite des ersten Tubus (120) und einen abgerundeten Abschnitt aufweist, wobei der zweite Tubus (130) ein Lumen (160) mit im Wesentlichen kreisförmigem Querschnitt aufweist;
Aufbringen eines Klebstoffes auf zumindest einen Abschnitt des ersten oder zweiten Tubus (130); und
Befestigen der Verbindungsfläche (138) des zweiten Tubus (130) an einer Außenseite des ersten Tubus (120), um dadurch den zweiten Tubus (130) mit dem ersten Tubus (120) zu verbinden, derart, dass die Verbindungsfläche (138) des zweiten Tubus (130), die an die Außenseite des ersten Tubus (120) angepasst ist, einen Verbindungssaum (140) zwischen den beiden Tuben (120, 130) erzeugt.

6. Verfahren nach Anspruch 5, wobei der Schritt des Aufbringens eines Klebstoffes den Schritt eines Aufbringens eines Klebstoffes auf die Verbindungsfläche (138) des zweiten Tubus (130) umfasst.

## Revendications

1. Canule (100) de trachéotomie comprenant :
un premier tube (120) de section transversale circulaire; et
un deuxième tube (130) de section transversale extérieure en forme de D;
le premier tube (120) et le deuxième tube (130) étant liés ensemble, et le deuxième tube (130) ayant une lumière (160) de section transversale sensiblement circulaire.

2. Tube (100) de trachéotomie suivant la revendication 1, dans lequel le deuxième tube (130) a une surface (138) de liaison qui se conforme à la forme de l'extérieur du premier tube (120).

3. Tube (100) de trachéotomie suivant la revendication 1 ou 2, dans lequel le premier tube (120) a un diamètre intérieur de l'ordre de 5,0 mm à 10,0 mm, et le deuxième tube (130) a un diamètre intérieur de l'ordre de 0,075 mm à 0,080 mm.

4. Tube (100) de trachéotomie suivant l'une quelconque des revendications précédentes, comprenant
un coussin (150) gonflable entourant le premier tube (120) à proximité de l'extrémité distale du premier tube (120);
le deuxième tube (130) étant lié au premier tube (120) de l'extrémité proximale du premier tube (120) à juste au-dessus du coussin (150) gonflable, ayant une ouverture formée en un emplacement juste au-dessus du coussin (150), qui permet, en utilisation, d'administrer des médicaments à un patient par le tube (100) de trachéotomie et qui permet aussi, en utilisation, d'aspirer des sécrétions d'un patient par le tube (100) de trachéotomie.

5. Procédé de fabrication d'un tube (100) de trachéotomie, qui consiste à prévoir un premier tube (120) ayant une section transversale cylindrique;
à prévoir un deuxième tube (130) ayant une section transversale extérieure en forme de D, de façon à ce que le deuxième tube (130) ait une surface (138) de liaison à l'extérieur du premier tube (120) et une partie arrondie, le deuxième tube (130) ayant une lumière (160) de section transversale sensiblement circulaire;
à appliquer un adhésif sur au moins partie de l'un du premier ou du deuxième tube (130); et
à fixer la surface (138) de liaison du deuxième tube (130) à l'extérieur du premier tube (120) en liant ainsi le deuxième tube (130) au premier tube (120) de façon à ce que la surface (138) de liaison du deuxième tube (130), qui se conforme à l'extérieur du premier tube (120), crée une ligne (140) de jonction entre les deux tubes (120, 130).

6. Procédé suivant la revendication 5, dans lequel le stade d'application d'un adhésif comprend le stade d'application d'un adhésif à la surface (138) de liaison du deuxième tube (130).
